# EUROPEAN PATENT SPECIFICATION

(11) **EP 2 559 433 B1**
(45) Date of publication and mention of the grant of the patent: **19.11.2014**
(21) Application number: 11177784.3
(22) Date of filing: 17.08.2011
(51) Int. Cl.: A61K 31/353, A61K 36/06

(54) **Method for manufacturing a composition for lowering blood lipid and elevating high-density lipoprotein.**
Herstellungsverfahren für eine Zusammensetzung zum Senken des Blutfetts und zum Erhöhen des hochdichten Lipoproteins.
Procédé de fabriquer une composition pour réduire les lipides sanguins et augmenter les lipoprotéines.

(43) Date of publication of application: 20.02.2013
(73) Proprietor: Sunway Biotech Co., Ltd., Taipei City 11494 (TW)
(72) Inventor: Pan, Tzu-Ming, 11494 Taipei City (TW); Lee, Chun-Lin, 11494 Taipei City (TW); Wu, Cheng-Lun, 11494 Taipei City (TW)
(74) Representative: Becker Kurig Straus

(56) References cited:
- US-A1- 2010 273 241
- LEE CHUN-LIN ET AL: "Monascin and Ankaflavin Act as Novel Hypolipidemic and High-Density Lipoprotein Cholesterol-Raising Agents in Red Mold Dioscorea", JOURNAL OF AGRICULTURAL AND FOOD CHEMISTRY, vol. 58, no. 16, August 2010 (2010-08), pages 9013-9019, XP002667414, ISSN: 0021-8561
- LEE C-L ET AL: "Enhanced hypolipidemic effect and safety of red mold dioscorea cultured in deep ocean water", JOURNAL OF AGRICULTURAL AND FOOD CHEMISTRY 20110810 AMERICAN CHEMICAL SOCIETY USA, vol. 59, no. 15, 10 August 2011 (2011-08-10), pages 8199-8207, XP002667415, DOI: DOI:10.1021/JF201948V
- CHUN-LIN LEE ET AL: "Monascus fermentation of dioscorea for increasing the production of cholesterol-lowering agent-monacolin K and antiinflammation agent-monascin", APPLIED MICROBIOLOGY AND BIOTECHNOLOGY, SPRINGER, BERLIN, DE, vol. 72, no. 6, 28 March 2006 (2006-03-28) , pages 1254-1262, XP019441683, ISSN: 1432-0614, DOI: 10.1007/S00253-006-0404-8
- DATABASE WPI Week 199736 Thomson Scientific, London, GB; AN 1997-385850 XP002667416, & CN 1 110 560 A (SHIXIN MEDICINE TECHNOLOGY DEV CENT CHEN) 25 October 1995 (1995-10-25)
- DATABASE WPI Week 199423 Thomson Scientific, London, GB; AN 1994-184166 XP002667417, & CN 1 075 875 A (UNIV BEIJING) 8 September 1993 (1993-09-08)
- DATABASE WPI Week 200335 Thomson Scientific, London, GB; AN 2003-364094 XP002667418, & CN 1 393 565 A (LIU D) 29 January 2003 (2003-01-29)
- DATABASE EMBASE [Online] ELSEVIER SCIENCE PUBLISHERS, AMSTERDAM, NL; January 2002 (2002-01), FU J -Y ET AL: "Effect of Monascin on serum lipid and body weight of experimental rats with hyperlipemia", XP002667465, Database accession no. EMB-2006540490 & CHINESE JOURNAL OF CLINICAL REHABILITATION 200201 CN, vol. 6, no. 1, January 2002 (2002-01), page 57+59, ISSN: 1671-5926
- DATABASE WPI Week 201113 Thomson Scientific, London, GB; AN 2010-P32505 XP002667420, & CN 101 864 191 A (UNIV FUZHOU) 20 October 2010 (2010-10-20)
- MANCHAND P S ET AL: "ISOLATION AND STRUCTURE OF ANKAFLAVIN: A NEW PIGMENT FROM MONASUCS ANKA", PHYTOCHEMISTRY, PERGAMON PRESS, GB, vol. 12, 1 January 1973 (1973-01-01), XP009025764, ISSN: 0031-9422, DOI: 10.1016/0031-9422(73)80470-4
- CHEN F C ET AL: "THE CHEMISTRY OF FUNGI. PART LXIV. THE STRUCTURE OF MONASCIN: THE RELATIVE STEREOCHEMSITRY OF THE AZAPHILONES", JOURNAL OF THE CHEMICAL SOCIETY, CHEMICAL SOCIETY, LETCHWORTH; GB, 1 January 1971 (1971-01-01), pages 3577-3579, XP009025762, ISSN: 0368-1769
- FIELDING B C ET AL: "THE CHEMISTRY OF FUNGI. PART XXXIX. THE STRUCTURE OF MONASCIN", JOURNAL OF THE CHEMICAL SOCIETY, CHEMICAL SOCIETY, LETCHWORTH; GB, 1 January 1961 (1961-01-01), pages 4579-4589, XP009025766, ISSN: 0368-1769, DOI: 10.1039/JR9610004579
- DATABASE WPI Week 199727 Thomson Scientific, London, GB; AN 1997-290194 XP002667421, & CN 1 103 891 A (JIANGMEN BIOTECHNOLOGY DEV CENT GUANGDON) 21 June 1995 (1995-06-21)
- DATABASE WPI Week 200901 Thomson Scientific, London, GB; AN 2009-A01890 XP002667422, & CN 101 255 168 A (INST AGRO FOOD SCI&TECHNOLOGY CHINESE) 3 September 2008 (2008-09-03)

## Description

### BACKGROUND OF THE INVENTION

### 1. Field of the Invention

The present invention relates methods for manufacturing a composition for lowering blood lipid and elevating high-density lipoprotein, and more particularly to the manufacture of a composition containing monascin or ankaflavin, or a combination thereof, for lowering blood lipid and elevating high-density lipoprotein.

### 2. Description of the Prior Art

The content of cholesterol in blood is closely related to the incidence of cardiovascular diseases. According to previous studies, dyslipidemia plays an important role in the cardiovascular diseases, thus the concentration of cholesterol in blood can be a well predictor for the cardiovascular diseases. When the concentration of serum cholesterol is more than 200 mg/dL, the probability of dying of coronary heart disease is raised rapidly. A person who has high content of cholesterol should have high morbidity rate of coronary heart disease and high death rate, but if the content of cholesterol is lowered by the treatment of a medical or a non-medical process, the incidence rate of the cardiovascular diseases caused by coronary sclerosis can be decreased significantly.

In recent years, health foods are developed vigorously, and multi-functional *Monascus* fermented products are gradually paid attention. In Asia, the application of *Monascus* species in food and medicine has thousand years of history.

The important secondary metabolites of *Monascus* species include: (1) a group of pigments, including red pigments (rubropunctamine and monascorubramine), yellow pigments (ankaflavin and monascin) and orange pigments (rubropunctanin and monascorubrin); (2) cholesterol-lowering substance, such as monacolin K (also called as lovastatin, mevinolin and mevacor); (3) blood pressure lowering substance, such as γ-aminobutyric acid (GABA); and (4) antioxidants, including dimerumic acid and 3-hydoxy-4-methoxy- benzoic acid.

In the above important secondary metabolites of *Monascus* species, owing to the monacolin K can reduce the activity of hydroxyl methylglutaryl-CoA reductase (HMG-CoA reductase), which is a key enzyme in the process of cholesterol biosynthesis, the monacolin K has a significantly effect for lowering cholesterol.

On the other hand, cholesterol in blood can be divided into two categories according to their density, including High Density Lipoprotein cholesterol (HDL-C) and Low Density Lipoprotein -Cholesterol (LDL-C). Many researches indicate that HDL-C is a kind of good cholesterol, and LDL-C is bad cholesterol. High concentration of HDL-C and low concentration of LDL-C are helpful in decreasing the incidence of cardiovascular disease and atherosclerosis.

Owing to total cholesterol (TC) is composed of HDL-C, LDL-C, triglyceride (TG), and other lipoprotein cholesterols, the amount of HDL-C and LDL-C can be substantially increased in company with the elevation of the concentration of TC. Most researches illustrate that after the concentration of TC being lowered by cholesterol-lowering drugs, the concentration of HDL-C is also decreased greatly. However, if the concentration of HDL-C can be maintained or elevated, the prevention of cardiovascular diseases can be achieved.

However, although the above-mentioned monacolin K has the effect of lowering cholesterol, the concentration of HDL-C can not be elevated by monacolin K, and the effect of preventing cardiovascular diseases and atherosclerosis by monacolin K is very limited.

In view of this, it is necessary to provide a novel composition and a method for manufacturing the same, wherein the composition has effects of lowering blood lipid and elevating the concentration of HDL-C simultaneously, so as to decrease the incidence of cardiovascular diseases and atherosclerosis effectively.

The following prior art documents describe methods for manufacturing ankaflavin/ monascin compositions:
DATABASE WPI, Week 201113 Thomson Scientific, London, GB; AN 2010-P32505, & CN 101 864 191 A (UNIV FUZHOU) 20 October 2010.
FIELDING B C ET AL: "THE CHEMISTRY OF FUNGI. PART XXXIX. THE STRUCTURE OF MONASCIN", JOURNAL OF THE CHEMICAL SOCIETY, CHEMICAL SOCIETY, LETCHWORTH; GB, 1 January 1961, pages 4579-4589.
DATABASE WPI, Week 199727 Thomson Scientific, London, GB; AN 1997-290194 XP002667421, & CN 1 103 891 A (JIANGMEN BIOTECHNOLOGY DEV CENT GUANGDON) 21 June 1995.

### SUMMARY OF THE INVENTION

In view of the above shortcomings of the prior art, the inventors of the present invention resorted to past experience, imagination, and creativity, performed experiments and researches repeatedly, and eventually devised the present invention, a composition for lowering blood lipid and elevating high-density lipoprotein and a method for manufacturing the same.

The major objective of the present invention is to provide a method of manufacturing a composition for lowering blood lipid and elevating high-density lipoprotein according to claim 1 which comprises monascin and can lower the concentration of blood lipid and elevate the concentration of high-density lipoprotein effectively.

According to the above objective, the present invention provides a composition for lowering blood lipid and elevating high-density lipoprotein, wherein the composition comprises monascin, which is a *Monascus* yellow pigment and extracted from a *Monascus* fermented product.

Another objective of the present invention is to provide the method for manufacturing a composition for lowering blood lipid and elevating high-density lipoprotein, by a series of extraction processes, a composition containing monascin can be obtained, and the composition can lower the concentration of blood lipid and elevate the concentration of high-density lipoprotein effectively.

According to the above objective, the present invention provides a method for manufacturing a composition for lowering blood lipid and elevating high-density lipoprotein, wherein the method comprises the steps of: (1) providing a *Monascus* fermented product; (2) treating the *Monascus* fermented product with acetone for three times; (3) elevating the concentration of the product obtained from the previous step by a process of decompress concentration in a specific temperature range; (4) separating a pigment fraction from the product obtained from the previous step by a silica gel column chromatography; (5) separating a yellow pigment fraction from the pigment fraction by a Sephadex LH-20 column chromatography; (6) separating a fraction containing monascin and ankaflavin from the yellow pigment fraction by the silica gel column chromatography; and (7) separating monascin from the fraction containing monascin and ankaftavin obtained from the previous step by a preparative high performance liquid chromatography (pre-HPLC).

Further objective of the present invention is to provide a method of manufacturing a composition for lowering blood lipid and elevating high-density lipoprotein according to claim 3 which comprises ankaflavin and can lower the concentration of blood lipid and elevate the concentration of high-density lipoprotein effectively.

According to the above objective, the present invention provides a composition for lowering blood lipid and elevating high-density lipoprotein, wherein the composition comprises ankaflavin, which is a *Monascus* yellow pigment and extracted from a *Monascus* fermented product.

Further objective of the present invention is to provide the method for manufacturing a composition for lowering blood lipid and elevating high-density lipoprotein, by a series of extraction processes, a composition containing ankaflavin can be obtained, and the composition can lower the concentration of blood lipid and elevate the concentration of high-density lipoprotein effectively.

According to the above objective, the present invention provides a method for manufacturing a composition for lowering blood lipid and elevating high-density lipoprotein, wherein the method comprises the steps of: (1) providing a *Monascus* fermented product; (2) treating the *Monascus* fermented product with acetone for three times; (3) elevating the concentration of the product obtained from the previous step by a process of decompress concentration in a specific temperature range; (4) separating a pigment fraction from the product obtained from the previous step by a silica gel column chromatography; (5) separating a yellow pigment fraction from the pigment fraction by a Sephadex LH-20 column chromatography; (6) separating a fraction containing monascin and ankaflavin from the yellow pigment fraction by the silica gel column chromatography; and (7) separating ankaflavin from the fraction containing monascin and ankaflavin obtained from the previous step by a pre-HPLC.

Further objective of the present invention is to provide a method of manufacturing a composition for lowering blood lipid and elevating high-density lipoprotein according to claim 5 which comprises monascin and ankaflavin and can lower the concentration of blood lipid and elevate the concentration of high-density lipoprotein effectively.

According to the above objective, the present invention provides a composition for lowering blood lipid and elevating high-density lipoprotein, wherein the composition comprises monascin and ankaflavin, which are both *Monascus* yellow pigments and extracted from a *Monascus* fermented product.

Further objective of the present invention is to provide the method for manufacturing a composition for lowering blood lipid and elevating high-density lipoprotein, by a series of extraction processes, a composition containing monascin and ankaflavin can be obtained, and the composition can lower the concentration of blood lipid and elevate the concentration of high-density lipoprotein effectively.

According to the above objective, the present invention provides a method for manufacturing a composition for lowering blood lipid and elevating high-density lipoprotein, wherein the method comprises the steps of: (1) providing a *Monascus* fermented product; (2) treating the *Monascus* fermented product with acetone for three times; (3) elevating the concentration of the product obtained from the previous step by a process of decompress concentration in a specific temperature range; (4) separating a pigment fraction from the product obtained from the previous step by a silica gel column chromatography; (5) separating a yellow pigment fraction from the pigment fraction by a Sephadex LH-20 column chromatography; (6) separating a fraction containing monascin and ankaflavin from the yellow pigment fraction by the silica gel column chromatography; (7) separating monascin and ankaflavin respectively from the fraction containing monascin and ankaflavin obtained from the previous step by a pre-HPLC; and (8) mixing the monascin and the ankaflavin according to a specific ratio.

### BRIEF DESCRIPTION OF THE DRAWINGS

For a complete understanding of the aspects, structures and techniques of the invention, reference should be made to the following detailed description and accompanying drawings wherein:
FIG. 1 is a flow chart diagram of a method for manufacturing a *Monascus* fermented product of the present invention;
FIG. 2 is a flow chart diagram of a method for manufacturing a composition for lowering blood lipid and elevating high-density lipoprotein according to a first preferred embodiment of the present invention;
FIG. 3 is a flow chart diagram of detailed steps of step 104 according to the first preferred embodiment of the present invention;
FIG. 4 is a flow chart diagram of detailed steps of step 105 according to the first preferred embodiment of the present invention;
FIG. 5 is a flow chart diagram of detailed steps of step 106 according to the first preferred embodiment of the present invention;
FIG. 6 is a flow chart diagram of detailed steps of step 107 according to the first preferred embodiment of the present invention;
FIG. 7 is a flow chart diagram of a method for manufacturing a composition for lowering blood lipid and elevating high-density lipoprotein according to a second preferred embodiment of the present invention;
FIG. 8 is a flow chart diagram of detailed steps of step 207 according to the second preferred embodiment of the present invention;
FIG. 9 is a flow chart diagram of a method for manufacturing a composition for lowering blood lipid and elevating high-density lipoprotein according to a third preferred embodiment of the present invention;
FIG. 10 is a flow chart diagram of detailed steps of step 307 according to the third preferred embodiment of the present invention;
FIG. 11 is a diagram of quantitative results of a lipid plaque staining experiment; and
FIG. 12 is a comparative diagram for the effect of the inhibition of cholesterol by monascin, ankaflavin and monacolin K with the same concentration.

### DETAILED DESCRIPTION OF THE PREFERRED EMBODIMENTS

To achieve the foregoing objectives and effects, the inventors utilize a *Monascus* fermented product as an extraction substrate, and extract the *Monascus* fermented product by a series of specific processes, under constant tests and regulations, thus achieving a composition for lowering blood lipid and elevating high-density lipoprotein and a method for manufacturing the same of the present invention. Hereinafter, the composition for lowering blood lipid and elevating high-density lipoprotein and the method for manufacturing the same according to a first, a second and a third preferred embodiment of the present invention are described in detail to illustrate the ingredients of the composition and the method for manufacturing the same of the present invention.

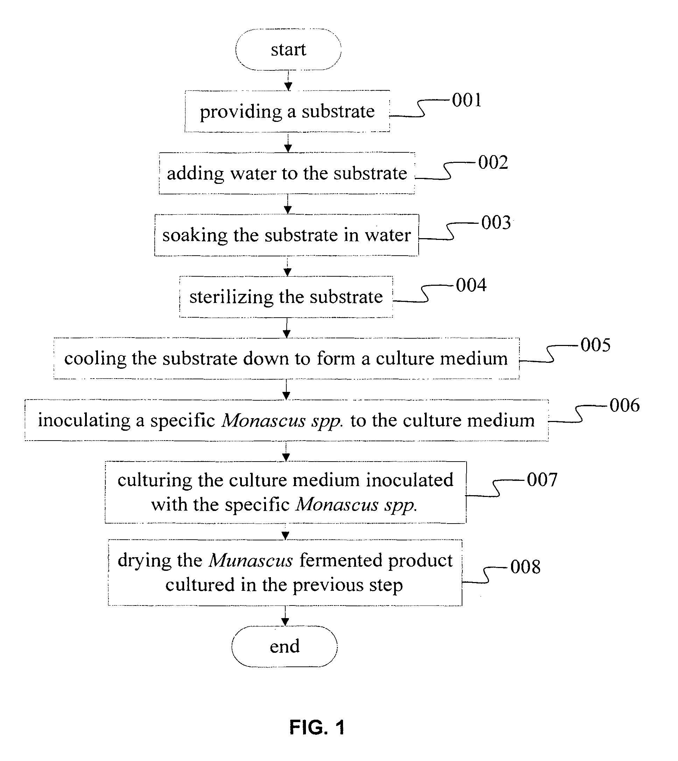

Before introducing the preferred embodiments of the present invention, the manufacturing method of a *Monascus* fermented product should be illustrated first. Referring to FIG. 1, which is a flow chart diagram of the method for manufacturing the *Monascus* fermented product of the present invention. The method comprises the steps of: (step 001) providing a substrate, which can be rice or dioscorea, wherein the water content of the substrate should be below 15%, and the optimal water content is 7%; (step 002) adding water to the substrate to make the ratio of the substrate to water be 1:0.5% ∼ 1:1.5%, wherein the optimal ratio is 1:0.75%; (step 003) soaking the substrate in water, wherein the soaking time is 0∼60 min, and the optimal time is 30 min; (step 004) sterilizing the substrate, wherein the sterilization condition is 121°C for 10∼60 min; (step 005) cooling the substrate down to form a culture medium; (step 006) inoculating a specific *Monascus spp.* to the culture medium, wherein the specific *Monascus spp.* can be *Monascus purpureus*; (step 007) culturing the culture medium inoculated with the specific *Monascus spp*., wherein the culture medium is cultured under a temperature of 25∼37°C, a humidity of 50∼80%, and for 8∼20 days, and the optimal culture condition is the temperature of 30, the humidity of 60%, and for 10 days; (step 008) drying the *Munascus* fermented product cultured in the previous step to make the *Munascus* fermented product have a water content below 15%, wherein the optimal water content is 6%. By the above processes, the Monascus fermented product is manufactured.

In the method for manufacturing the *Monascus* fermented product of the present invention, the strains of *Monascus purpureus* can be selected from *Monascus purpureus* NTU 568 or Monascus purpureus PAN 790.

*Monascus purpureus* NTU 568 is deposited in Agricultural Research Culture Collection (NRRL) in November 13, 2009, and the given accession number is NRRL 50338. The characteristics of *Monascus purpureus* NTU 568 includes rapid growth, strong ability of starch hydrolysis, and strong ability of metabolites production. The basic culture manner of *Monascus purpureus* NTU 568 is to be cultured with a medium containing 2% of rice powder, the best culture temperature is 30°C, the best culture time is 48 hours, the best culture pressure is 1 atm, and the growth of *Monascus purpureus* NTU 568 is oxygen dependent.

*Monascus purpureus* PAN 790 is deposited in Agricultural Research Culture Collection (NRRL) in November 13, 2009, and the given accession number is NRRL 50337. The characteristics of *Monascus purpureus* PAN 790 includes slow growth, being able to produce large amount of monacolin K, and being mutated in and cloned from red mold rice. The culture medium for *Monascus purpureus* PAN 790 includes 2% of rice powder; the culture temperature is 30°C; the culture time is 48 hours; the culture pressure is 1 atm; the sterilization manner for *Monascus purpureus* PAN 790 is 121°C for 20 min; the pH value before sterilization is 5; the growth of *Monascus purpureus* PAN 790 is oxygen dependent; and the storage temperature for *Monascus purpureus* PAN 790 is 4°C.

Subsequently, the first preferred embodiment of the present invention is described in detail. The composition for lowering blood lipid and elevating high-density lipoprotein according to the first preferred embodiment of the present invention contains monascin, which is a *Monascus* yellow pigment, and extracted from the above-mentioned *Monascus* fermented product. Wherein an amount of the monascin taken by an adult is more than 2.4 mg per day, so that the concentration of blood lipid can be lowered and the concentration of the high-density lipoprotein in blood can be elevated effectively.

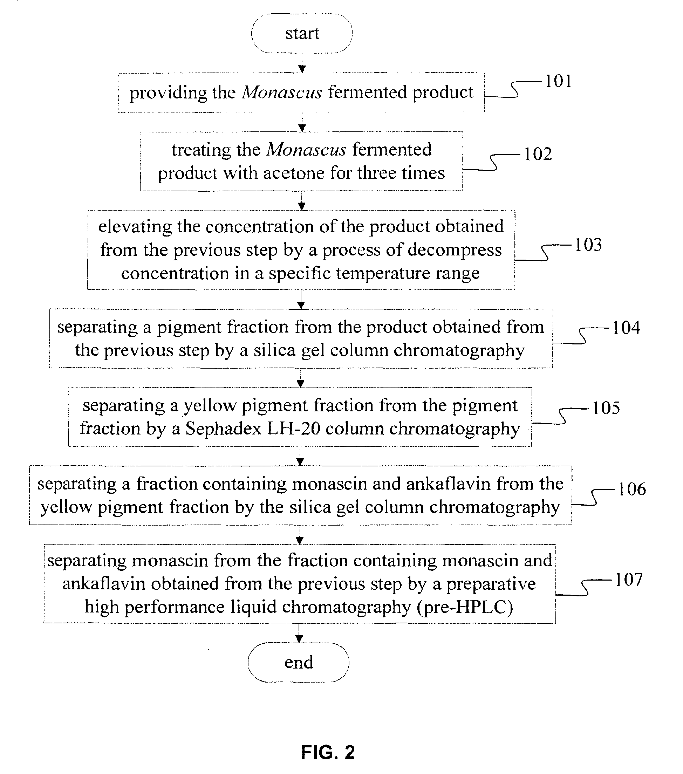

Referring to Fig. 2, which is a flow chart diagram of a method for manufacturing the composition for lowering blood lipid and elevating high-density lipoprotein according to the first preferred embodiment of the present invention. The method includes the steps of: (step 101) providing the *Monascus* fermented product; (step 102) treating the *Monascus* fermented product with acetone for three times, wherein the ratio of the *Monascus* fermented product to the acetone is 1:10 ∼ 1:50; (step 103) elevating the concentration of the product obtained from the previous step by a process of decompress concentration in a specific temperature range, wherein the specific temperature range is 40□ ∼60□; (step 104) separating a pigment fraction from the product obtained from the previous step by a silica gel column chromatography; (step 105) separating a yellow pigment traction from the pigment fraction by a Sephadex LH-20 column chromatography; (step 106) separating a fraction containing monascin and ankaflavin from the yellow pigment fraction by the silica gel column chromatography; and (step 107) separating monascin from the fraction containing monascin and ankaflavin obtained from the previous step by a preparative high performance liquid chromatography (pre-HPLC).

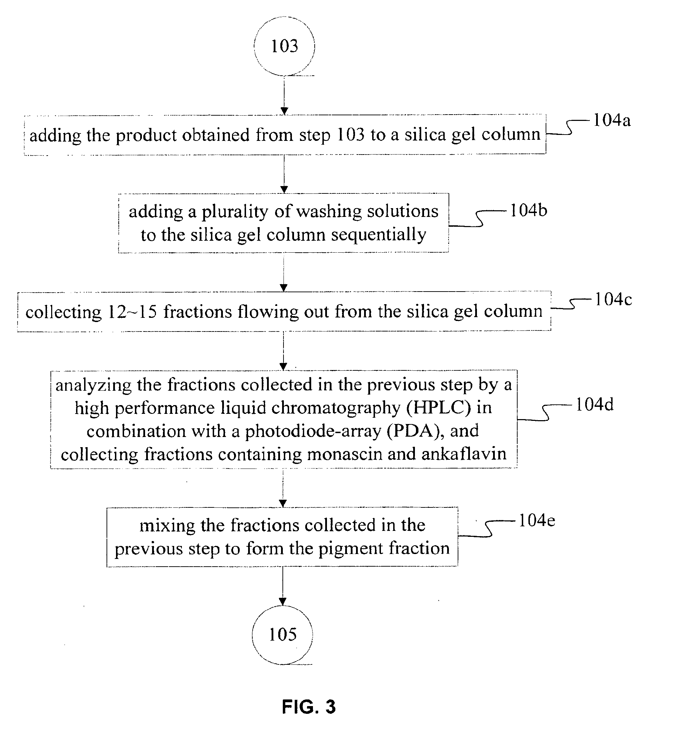

Referring to Fig. 3, which is a flow chart diagram of detailed steps of step 104 according to the first preferred embodiment of the present invention. The above step 104 can be subdivided into the following steps of: (step 104a) adding the product obtained from step 103 to a silica gel column; (step 104b) adding a plurality of washing solutions to the silica gel column sequentially, wherein the plurality of washing solutions are Hexane, Hexane : Ethanol = 9:1, Hexane : Ethanol = 8:2, Hexane : Ethanol = 7:3, Hexane : Ethanol = 1:1, and Ethanol sequentially; (step 104c) collecting 12∼15 fractions flowing out from the silica gel column, wherein the time period of each fraction flowing out from the silica gel column is 5∼10 min; (step 104d) analyzing the fractions collected in the previous step by a high performance liquid chromatography (HPLC) in combination with a photodiode-array (PDA), and collecting fractions containing monascin and ankaflavin; and (step 104e) mixing the fractions collected in the previous step to form the pigment fraction.

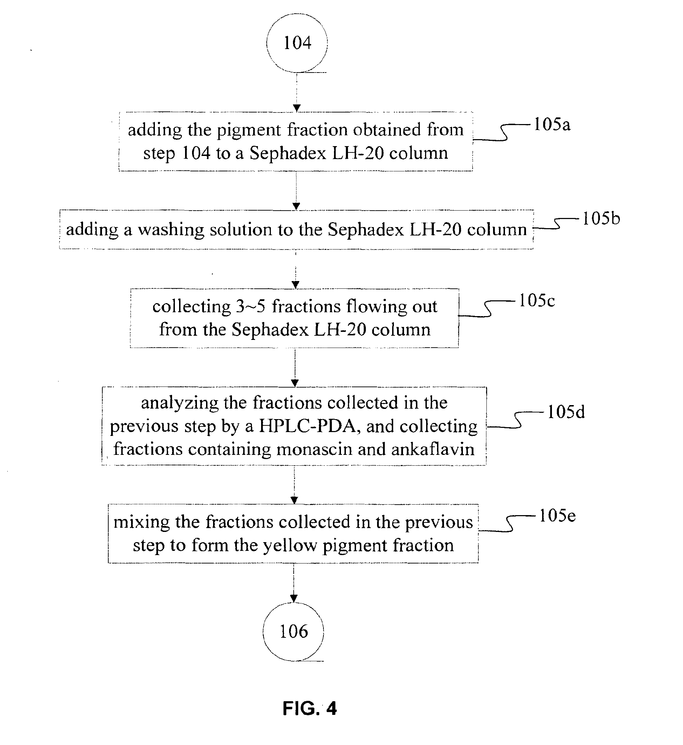

Referring to Fig. 4, which is a flow chart diagram of detailed steps of step 105 according to the first preferred embodiment of the present invention. The above step 105 can be subdivided into the following steps of: (step 105a) adding the pigment fraction obtained from step 104 to a Sephadex LH-20 column; (step 105b) adding a washing solution to the Sephadex LH-20 column, wherein the washing solution is Methanol : Acetonitrile = 9:1; (step 105c) collecting 3∼5 fractions flowing out from the Sephadex LH-20 column, wherein the time period of each fraction flowing out from the silica gel column is 5∼10 min; (step 105d) analyzing the fractions collected in the previous step by a HPLC-PDA, and collecting fractions containing monascin and ankaflavin; and (step 105e) mixing the fractions collected in the previous step to form the yellow pigment fraction.

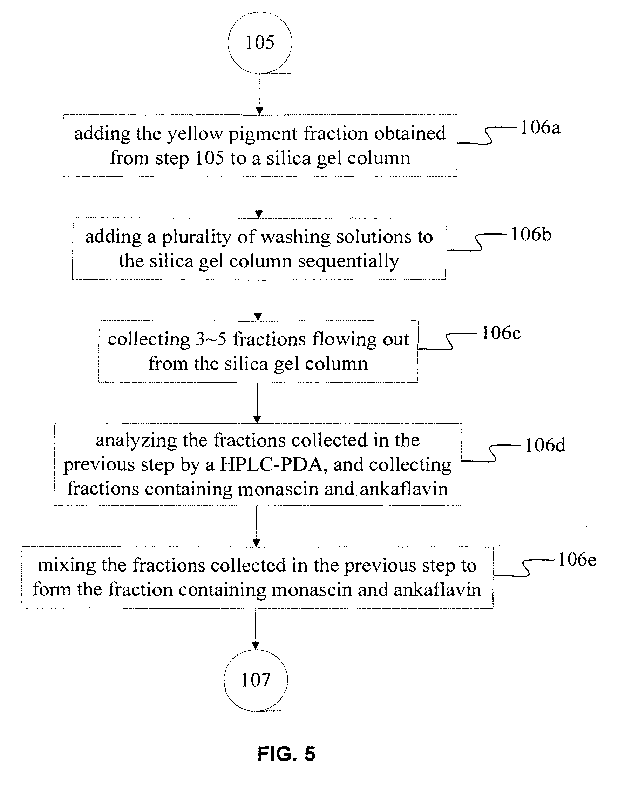

Referring to Fig. 5, which is a flow chart diagram of detailed steps of step 106 according to the first preferred embodiment of the present invention. The above step 106 can be subdivided into the following steps of: (step 106a) adding the yellow pigment fraction obtained from step 105 to a silica gel column; (step 106b) adding a plurality of washing solutions to the silica gel column sequentially, wherein the plurality of washing solutions are Dichloromethane : Ethanol = 95:5, Dichloromethane : Ethanol = 9:1, and Dichloromethane : Ethanol = 4:1 sequentially; (step 106c) collecting 3∼5 fractions flowing out from the silica gel column, wherein the time period of each fraction flowing out from the silica gel column is 5∼10 min; (step 106d) analyzing the fractions collected in the previous step by a HPLC-PDA, and collecting fractions containing monascin and ankaflavin; and (step 106e) mixing the fractions collected in the previous step to form the fraction containing monascin and ankaflavin.

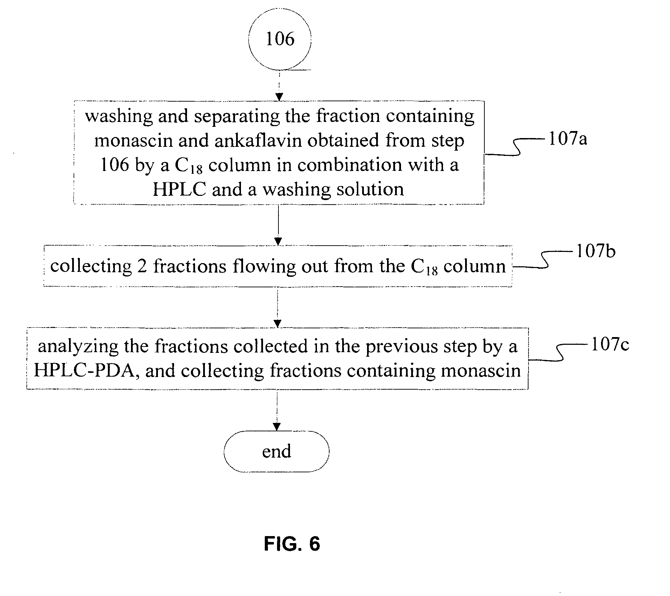

Referring to Fig. 6, which is a flow chart diagram of detailed steps of step 107 according to the first preferred embodiment of the present invention. The above step 107 can be subdivided into the following steps of: (step 107a) washing and separating the fraction containing monascin and ankaflavin obtained from step 106 by a C₁₈ column in combination with a HPLC and a washing solution, wherein the washing solution is Methanol : water = 85:15; (step 107b) collecting 2 fractions flowing out from the C₁₈ column, wherein the time period of each fraction flowing out from the silica gel column is 5∼10 min; and (step 107c) analyzing the fractions collected in the previous step by a HPLC-PDA, and collecting fractions containing monascin.

In the above steps 104d, 105d and 106d, the column applied in the HPLC process is C18 column (25 cm * 4.6 mm, i.d. = 5µm), the mobile phase is Acetonitrile : water : trifluoroacetate = 62:38:0.05, the speed of flow is 1 mL/min, and the UV absorption amount under 231 nm is detected with a multiwavelength detector (because of the curves of monascin and ankaflavin have peaks under 231 nm). Furthermore, standards of monascin and ankaflavin should be applied as comparison groups for comparing the peaks with the ones of analytes, so as to determine whether the analytes are monascin and ankaflavin or not.

Additionally, in the above-mentioned step 107c, the analysis conditions of HPLC process are almost the same as step 104d, step 105d and step 106d. However, the special aspect of step 107c is that monascin and ankaflavin are separated from each other by the property of polarity difference. Owing to the C₁₈ column can wash out the more polar ingredients at earlier time point, and monascin is more polar relative to ankaflavin, monascin can be washed out from C₁₈ column earlier than ankaflavin. By this property, monascin can be collected in the fractions washed out at earlier time point, and monascin in the fractions can be identified by comparing the washing time and the absorption wavelength with the monascin standard.

Subsequently, the second preferred embodiment of the present invention is described in detail. The composition for lowering blood lipid and elevating high-density lipoprotein according to the second preferred embodiment of the present invention contains ankaflavin, which is a *Monascus* yellow pigment, and extracted from the above-mentioned *Monascus* fermented product. Wherein an amount of the ankaflavin taken by an adult is more than 0.6 mg per day, so that the concentration of blood lipid can be lowered and the concentration of the high-density lipoprotein in blood can be elevated effectively.

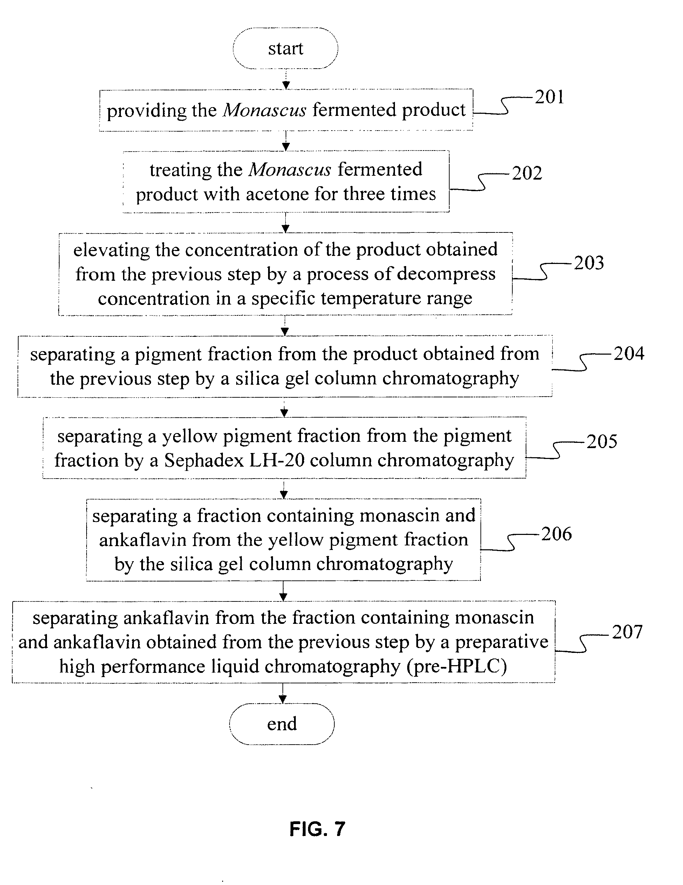

Referring to Fig. 7, which is a flow chart diagram of a method for manufacturing the composition for lowering blood lipid and elevating high-density lipoprotein according to the second preferred embodiment of the present invention. Steps 201∼206 of the second preferred embodiment are almost the same as the steps 101∼106, and thus the steps 201∼206 will not be described again here. The difference between the second preferred embodiment and the first preferred embodiment is that step 207 of the second preferred embodiment separates ankaflavin by the pre-HPLC process.

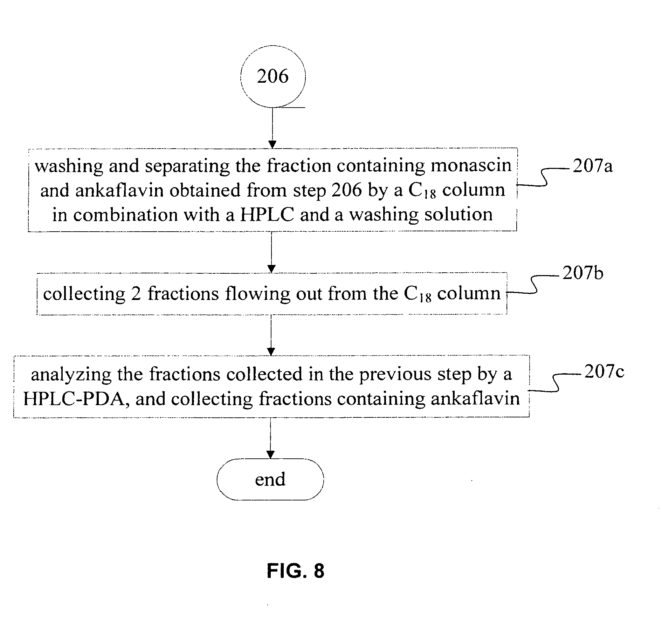

Referring to Fig. 8, which a flow chart diagram of detailed steps of step 207 according to the second preferred embodiment of the present invention. The above step 207 can be subdivided into the following steps of: (step 207a) washing and separating the fraction containing monascin and ankaflavin obtained from step 206 by a C₁₈ column in combination with a HPLC and a washing solution, wherein the washing solution in step (7.1) is Methanol : water = 85:15; (Step 207b) collecting 2 fractions flowing out from the C₁₈ column, wherein the time period of each fraction flowing out from the silica gel column is 5∼10 min; and (step 207c) analyzing the fractions collected in the previous step by a HPLC-PDA, and collecting fractions containing ankaflavin.

In the above-mentioned step 207c, monascin and ankaflavin are separated from each other by the property of polarity difference. Owing to the C₁₈ column can wash out the more polar ingredients at earlier time point, and monascin is more polar relative to ankaflavin, monascin can be washed out from C₁₈ column earlier than ankaflavin. By this property, ankaflavin can be collected in the fractions washed out at later time point, and ankaflavin in the fractions can be identified by comparing the washing time and the absorption wavelength with the ankaflavin standard.

Subsequently, the third preferred embodiment of the present invention is described in detail. The composition for lowering blood lipid and elevating high-density lipoprotein according to the third preferred embodiment of the present invention contains monascin and ankaflavin, which are both *Monascus* yellow pigments, and extracted from the above-mentioned *Monascus* fermented product. Wherein the content ratio of the monascin to the ankaflavin is in a range from 1:1 to 10:1, and the optimum content ratio of the monascin to the ankaflavin is 3.56:1. Furthermore, an amount of the monascin taken by an adult is more than 2.4 mg per day, and an amount of the ankaflavin taken by an adult is more than 0.6 mg per day, so that the concentration of blood lipid can be lowered and the concentration of the high-density lipoprotein in blood can be elevated effectively.

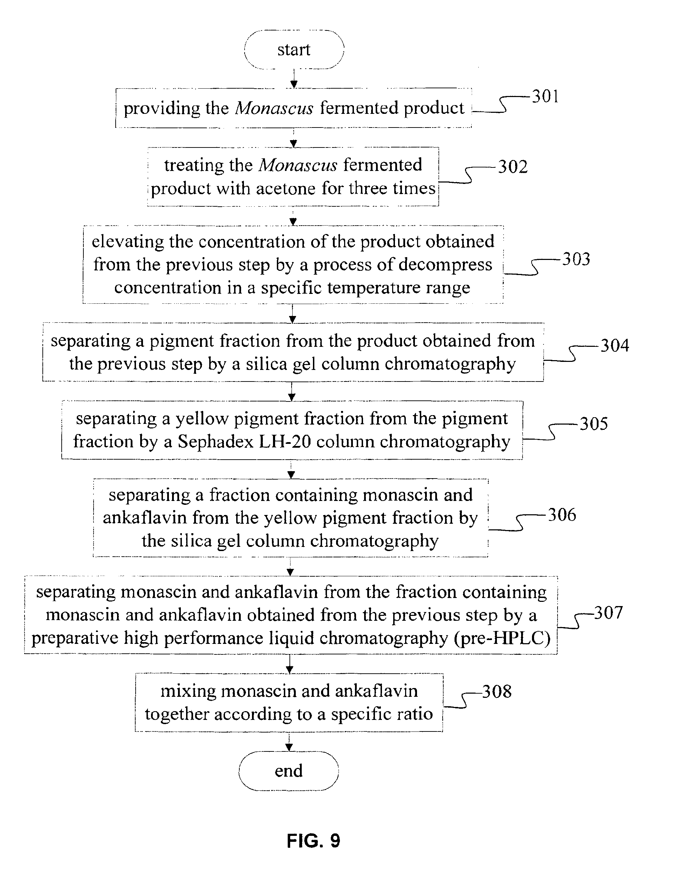

Referring to Fig. 9, which is a flow chart diagram of a method for manufacturing the composition for lowering blood lipid and elevating high-density lipoprotein according to the third preferred embodiment of the present invention. Steps 301∼306 of the third preferred embodiment are almost the same as the steps 101∼106 and 201∼206, and thus the steps 301∼306 will not be described again here. The difference between the third preferred embodiment and the first preferred embodiment (or second preferred embodiment) is that step 307 of the third preferred embodiment separates monascin and ankaflavin by the pre-HPLC process. Eventually, according to step 308, mixing monascin and ankaflavin together according to a specific ratio, wherein the specific ratio is in a range from 1:1 to 10:1, and the optimum value of the specific ratio is 3.56:1.

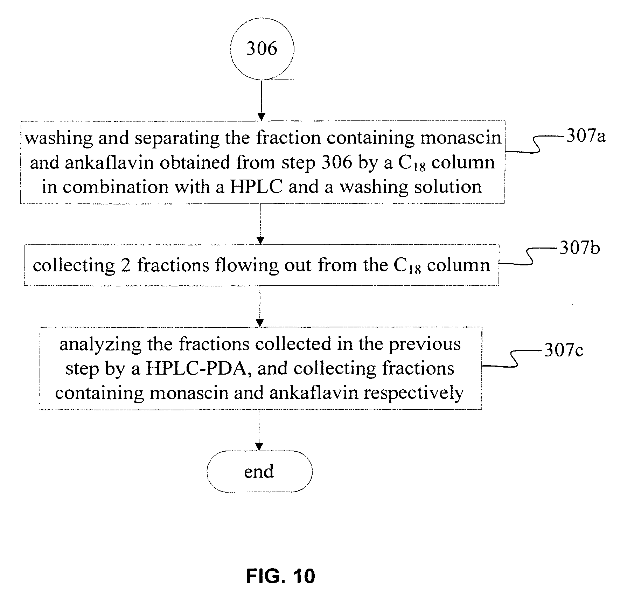

Referring to Fig. 10, which is a flow chart diagram of detailed steps of step 307 according to the third preferred embodiment of the present invention. The above step 307 can be subdivided into the following steps of: (step 307a) washing and separating the fraction containing monascin and ankaflavin obtained from step 306 by a C₁₈ column in combination with a HPLC and a washing solution, wherein the washing solution is Methanol : water = 85:15; (step 307b) collecting 2 fractions flowing out from the C₁₈ column, wherein the time period of each fraction flowing out from the C₁₈ column is about 5∼10 min; and (step 307c) analyzing the fractions collected in the previous step by a HPLC-PDA, and collecting fractions containing monascin and ankaflavin respectively.

In the above-mentioned step 307c, monascin and ankaflavin are separated from each other by the property of polarity difference. Owing to the C₁₈ column can wash out the more polar ingredients at earlier time point, and monascin is more polar relative to ankaflavin, monascin can be washed out from C₁₈ column earlier than ankaflavin. By this property, monascin can be collected in the fractions washed out at earlier time point, and ankaflavin can be collected in the fractions washed out at later time point. Furthermore, monascin and ankaflavin in the fractions can be identified by comparing the washing time and the absorption wavelength with the monascin and ankaflavin standards.

In order to prove that the compositions of the first, the second and the third preferred embodiments indeed have the effect of lowering blood lipid and elevating high-density lipoprotein, the compositions are analyzed and compared for their functions by several experiments as followings. Hereinafter, the composition of the first preferred embodiment is called as monascin (MS for abbreviation), and the amount of the monascin taken by an experimental animal is equivalent to 9.82 mg taken by an adult human per day; the composition of the second preferred embodiment is called as ankaflavin (AF for abbreviation), and the amount of the ankaflavin taken by an experimental animal is equivalent to 1.43 mg taken by an adult human per day; the composition of the third preferred embodiment is called as monascin + ankaflavin (MS+AF for abbreviation), and the amount of the monascin and ankaflavin taken by an experimental animal are equivalent to 9.82 mg and 1.43 mg taken by an adult human per day respectively. Additionally, a composition containing monacolin K is utilized as a comparison group, which is called as MK for abbreviation.

Firstly, the experiment utilizes hamsters feed with high cholesterol diet as experimental animals, which are then feed with MS, AF, MS+AF, and MK for 8 weeks, and then each of cholesterol parameters in blood of the hamsters are analyzed. The cholesterol parameters include total cholesterol (TC), triglyceride (TG), high density lipoprotein cholesterol (HDL-C), low density lipoprotein cholesterol (LDL-C), ratio of LDL-C to HDL-C, and malondialdehyde (MDA). The amount of the MK taken by the experimental animals is equivalent to 2.89 mg taken by an adult human per day. Additionally, a normal diet group is referred to as NOR, and a high cholesterol diet group is referred to as HC.

Referring to the following table 1 and table 2, wherein the table 1 illustrates the results of the above-mentioned animal experiments, and the table 2 illustrates the reducing rate or the increasing rate of MS, AF, MS+AF, and MK relative to HC. In the results of the reducing rate of TC, the groups of MS and AF both can lower the amount of TC in serum significantly, and the effect of MS and AF are better than that of MK; the group of MS+AF has the effect of lowering the amount of TC better than the other groups. In the results of the reducing rate of TG, groups of MS, AF and MK+AF can lower the amount of TG in serum significantly, and the effect of MS, AF and MS+AF are better than that of MK. In the results of the increasing rate of HDL-C, the groups of MS and AF both can increase the amount of TC in serum significantly, and the effect of MS and AF are better than that of MK; the group of MS+AF has the effect of increasing the amount of HDL-C better than the other groups. In the results of the reducing rate of LDL-C, although the group of MS has the effect almost the same as MK, the groups of AF and MS+AF have the effect of lowering the amount of LDL-C better than the MK group. In the results of the reducing rate of LDL/HDL, the effect of MS group is better than MK group, and the effects of AF and MS+AF are better than the other groups, thus monascin, ankaflavin and monascin+ankaflavin is more contributive to ameliorate the occurrence of cardiovascular diseases and atherosclerosis than monacolin K. In the results of the reducing rate of MDA, the groups of MS, AF and MS+AF can lower the amount of MDA in serum significantly, and the effects of the groups of MS, AF and MS+AF are all better than the MK group.

**Table 1**

| Group | TC content (mg/dL) | TG content (mg/dL) | HDL-C content (mg/dL) | LDL-C content (mg/dL) | LDL/HDL | MDA content (mg/dL) |
|---|---|---|---|---|---|---|
| NOR | 111.8 ±10.7 | 168.8 ±35.6 | 66.6 ±5.97 | 19.9 ±2.59 | 0.278 ±0.038 | 8.93 ±1.79 |
| HC | 236.5 ±18.9 | 226.3 ±76.5 | 98.1 ±8.89 | 68.3 ±9.75 | 0.569 ±0.093 | 11.80 ±1.85 |
| MK | 190.3 ±25.1 | 131.1 ±36.7 | 96.9 ±8.84 | 45.0 ±6.97 | 0.489 ±0.050 | 10.37 ±1.73 |
| MS | 165.8 ±10.6 | 82.8 ±9.0 | 114.2 ±9.36 | 45.1 ±336 | 0.416 ±0.022 | 8.62 ±1.45 |
| AF | 168.9 ±11.5 | 94.5 ±18.0 | 118.6 ±8.09 | 39.4 ±5.80 | 0.355 ±0.049 | 9.07 ±1.45 |
| MS+AF | 160.3 ±7.6 | 85.6 ±10.60 | 125.7 ±7.30 | 38.5 ±3.10 | 0.351 ±0.051 | 8.45 ±1.23 |

**Table 2**

| group | TC reducing rate (%) | TG reducing rate (%) | HDL-C increasing rate (%) | LDL-C reducing rate (%) | LDL/HDL reducing rate (%) | MDA reducing rate (%) |
|---|---|---|---|---|---|---|
| MK | 19.6 | 42.1 | -1.2 | 34.1 | 14.1 | 16.5 |
| MS | 29.9 | 63.4 | 14.1 | 33.9 | 26.9 | 40.1 |
| AF | 28.6 | 58.2 | 17.3 | 42.3 | 37.6 | 59.6 |
| MS+AF | 29.2 | 63.8 | 22.0 | 43.6 | 38.3 | 58.9 |

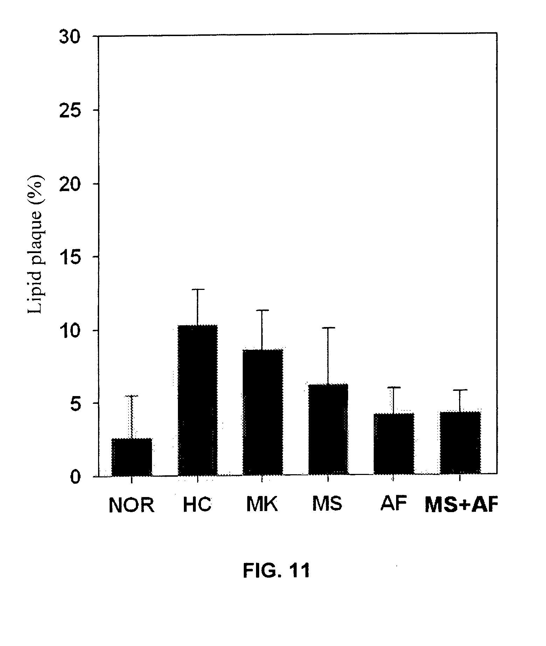

Another important indicator for evaluating atherosclerosis is the analysis of lipid plaque staining in arteries, and the results of lipid plaque staining can indicate that whether lipid plaque deposits on arterial vascular walls. Large amounts of lipid with over-oxidation can cause bulk deposition of foam cells, and then the vascular walls lose elasticity and large amounts of lipid plaque are deposited, so as to lead to atherosclerosis. Referring to Fig. 11, which is a diagram of quantitative results of the lipid plaque staining experiment. The results demonstrate that high cholesterol diet (HC group) for eight weeks may cause the experimental animals to deposit large amounts of lipid plaque on arterial vascular walls in the thoracic cavity, but the deposition of lipid plaque in the NOR group is less than HC group. Although the MK group (the amount of the MK taken is 2.89 mg taken by an adult human per day) can lower the amount of lipid plaque, the effect of the MK group is not as good as that of the MS (the amount of the MS taken is 9.82 mg taken by an adult human per day), AF (the amount of the AF taken is 1.43 mg taken by an adult human per day) and MS+AF groups (the content ratio of the monascin to the ankaflavin is 3.56:1); the effect of the AF group is better than that of the MS group, and the effect of the MS+AF group is better than that of the AF group.

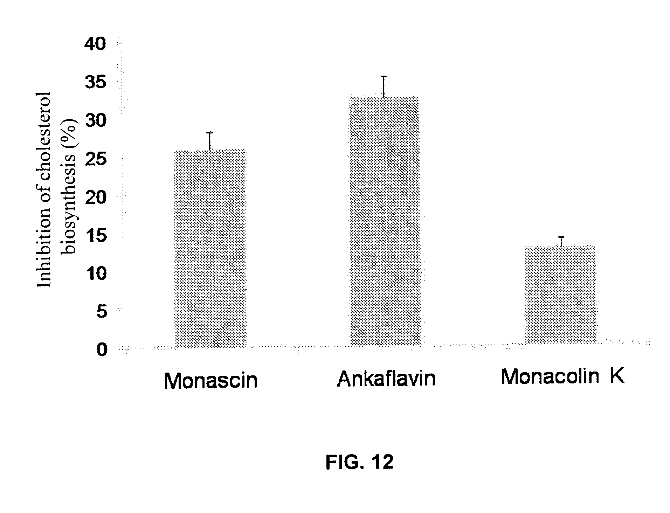

In order to compare the inhibitory effect of cholesterol biosynthesis by monascin, ankaflavin and monacolin K, the present invention applies a cell experiment and treats cells with monascin, ankaflavin and monacolin K with the same concentration (100 ppb) for researching the influence of these ingredients on the cholesterol biosynthesis. Referring to Fig. 12, which is a comparative diagram for the effect of the inhibition of cholesterol by monascin, ankaflavin and monacolin K with the same concentration. The results demonstrate that ankaflavin has the best effect of inhibiting the biosynthesis of cholesterol, followed by monascin, and monacolin K has the weakest effect. These results are consistent with the above animal experiments, that is ankaflavin and monascin have better effect on blood lipid lowering than monacolin K.

It can be known from the above experiments that monascin, ankaflavin and monascin+ankaflavin can reduce TC, TG, LDL-C, MDA, and lipid plaque significantly, and can elevate the content of HDL-C in serum; additionally, the effect of monascin+ankaflavin is more prominent than the effect of monascin or ankaflavin. Thus, the compositions according to the first, second and third preferred embodiments not only can reduce the content of cholesterol in serum, but also can elevate the concentration of HDL-C in serum, so as to have extreme contribution to lower the incidence of cardiovascular diseases and atherosclerosis.

By the detailed description of the overall structure and technical content of the present invention, the following advantages of the present invention can be derived:
1. Each of the compositions provided by the present invention all have the effect of lowering blood lipid and elevating high-density lipoprotein, thus these compositions can be utilized to prevent and treat cardiovascular diseases and atherosclerosis.
2. Through the manufacturing method provided by the present invention, each of the compositions with high purity of the present invention can be extracted, and can be applied to lower the incidence of cardiovascular diseases and atherosclerosis.
3. Each of the compositions of the present invention are extracted from *Monascus* fermented product, not synthesized artificially, thus the compositions can be regarded as natural food supplements, which not only can reduce the incidence of cardiovascular diseases and atherosclerosis, but also can avoid the production of side effects.

It should be understood that the embodiments of the present invention described herein are merely illustrative of the technical concepts and features of the present invention and are not meant to limit the scope of the invention. Those skilled in the art, after reading the present disclosure, will know how to practice the invention. Various variations or modifications can be made without departing from the spirit of the invention.

As a result of continued thinking about the invention and modifications, the inventors finally work out the designs of the present invention that has many advantages as described above. The present invention meets the requirements for an invention patent, and the application for a patent is duly filed accordingly. It is expected that the invention could be examined at an early date and granted so as to protect the rights of the inventors.

## Claims

1. A method for manufacturing a composition for lowering blood lipid and elevating high-density lipoprotein, wherein the method comprises the steps of:
(1) providing a *Monascus* fermented product;
(2) treating the *Monascus* fermented product with acetone for three times, wherein the ratio of the Monascus fermented product to the acetone is 1:10 ∼ 1:50;
(3) elevating the concentration of the product obtained from the previous step by a process of decompress concentration in a specific temperature range, wherein the specific temperature range is 40°C ∼60°C;
(4.1) adding the product obtained from step (3) to one silica gel column;
(4.2) adding a plurality of washing solutions to the silica gel column sequentially, wherein the plurality of washing solutions in the step (4.2) are Hexane, Hexane : Ethanol = 9:1, Hexane : Ethanol = 8:2, Hexane :Ethanol = 7:3, Hexane : Ethanol = 1:1, and Ethanol sequentially;
(4.3) collecting 12∼15 fractions flowing out from the silica gel column;
(4.4) analyzing the fractions collected in the previous step by one high performance liquid chromatography in combination with one photodiode-array (HPLC-PDA), and collecting fractions containing monascin and ankaflavin;
(4.5) mixing the fractions collected in the previous step to form a pigment fraction;
(5) separating a yellow pigment fraction from the pigment fraction by a Sephadex LH-20 column chromatography;
(6.1) adding the yellow pigment fraction obtained from step (5) to one silica gel column;
(6.2) adding a plurality of washing solutions to the silica gel column sequentially, wherein the plurality of washing solutions in step (6.2) are Dichloromethane : Ethanol = 95:5, Dichloromethane : Ethanol = 9:1, and Dichloromethane : Ethanol = 4:1 sequentially;
(6.3) collecting 3∼5 fractions flowing out from the silica gel column;
(6.4) analyzing the fractions collected in the previous step by one HPLC-PDA, and collecting fractions containing monascin and ankaflavin;
(6.5) mixing the fractions collected in the previous step to form a specific fraction containing monascin and ankaflavin;
(7.1) washing and separating the specific fraction containing monascin and ankaflavin obtained from step (6) by a C₁₈ column in combination with one high performance liquid chromatography (HPLC) and a washing solution, wherein the washing solution in step (7.1) is Methanol : water = 85:15;
(7.2) collecting 2 fractions flowing out from the C₁₈ column; and
(7.3) analyzing the fractions collected in the previous step by one HPLC-PDA, and collecting fractions containing monascin.

2. The method for manufacturing the composition for lowering blood lipid and elevating high-density lipoprotein according to claim 1, wherein the step (5) further comprises the steps of:
(5.1) adding the pigment fraction obtained from step (4) to one Sephadex LH-20 column;
(5.2) adding a washing solution to the Sephadex LH-20 column;
(5.3) collecting 3∼5 fractions flowing out from the Sephadex LH-20 column;
(5.4) analyzing the fractions collected in the previous step by one HPLC-PDA, and collecting fractions containing monascin and ankaflavin; and
(5.5) mixing the fractions collected in the previous step to form the yellow pigment fraction;
wherein the washing solution in step (5.2) is Methanol : Acetonitrile = 9:1.

3. A method for manufacturing a composition for lowering blood lipid and elevating high-density lipoprotein, wherein the method comprises the steps of:
(1) providing a *Monascus* fermented product;
(2) treating the *Monascus* fermented product with acetone for three times, wherein the ratio of the *Monascus* fermented product to the acetone is 1:10 ∼ 1:50;
(3) elevating the concentration of the product obtained from the previous step by a process of decompress concentration in a specific temperature range, wherein the specific temperature range is 40°C∼60°C ;
(4.1) adding the product obtained from step (3) to a silica gel column;
(4.2) adding a plurality of washing solutions to the silica gel column sequentially, wherein the plurality of washing solutions in step (4.2) are Hexane, Hexane : Ethanol = 9:1, Hexane : Ethanol = 8:2, Hexane :Ethanol = 7:3, Hexane : Ethanol = 1:1, and Ethanol sequentially;
(4.3) collecting 12∼15 fractions flowing out from the silica gel column;
(4.4) analyzing the fractions collected in the previous step by o one high performance liquid chromatography in combination with one photodiode-array (HPLC-PDA), and collecting fractions containing monascin and ankaflavin; and
(4.5) mixing the fractions collected in the previous step to form a pigment fraction;
(5) separating a yellow pigment fraction from the pigment fraction by a Sephadex LH-20 column chromatography;
(6.1) adding the yellow pigment fraction obtained from step (5) to one silica gel column;
(6.2) adding a plurality of washing solutions to the silica gel column sequentially, wherein the plurality of washing solutions in step (6.2) are Dichloromethane : Ethanol = 95:5, Dichloromethane : Ethanol = 9:1, and Dichloromethane : Ethanol = 4:1 sequentially;
(6.3) collecting 3-5 fractions flowing out from the silica gel column;
(6.4) analyzing the fractions collected in the previous step by one HPLC-PDA, and collecting fractions containing monascin and ankaflavin;
(6.5) mixing the fractions collected in the previous step to form a specific fraction containing monascin and ankaflavin;
(7.1) washing and separating the specific fraction containing monascin and ankaflavin obtained from step (6) by a C₁₈ column in combination with one high performance liquid chromatography (HPLC) and a washing solution, wherein the washing solution in step (7.1) is Methanol : water = 85:15;
(7.2) collecting 2 fractions flowing out from the C₁₈ column; and
(7.3) analyzing the fractions collected in the previous step by one HPLC-PDA, and collecting fractions containing ankaflavin.

4. The method for manufacturing the composition for lowering blood lipid and elevating high-density lipoprotein according to claim 3, wherein the step (5) further comprises the steps of:
(5.1) adding the pigment fraction obtained from step (4) to one Sephadex LH-20 column;
(5.2) adding a washing solution to the Sephadex LH-20 column;
(5.3) collecting 3-5 fractions flowing out from the Sephadex LH-20 column;
(5.4) analyzing the fractions collected in the previous step by one HPLC-PDA, and collecting fractions containing monascin and ankaflavin; and
(5.5) mixing the fractions collected in the previous step to form the yellow pigment fraction;
wherein the washing solution in step (5.2) is Methanol : Acetonitrile =9:1.

5. A method for manufacturing a composition for lowering blood lipid and elevating high-density lipoprotein, wherein the method comprises the steps of:
(1) providing a *Monascus* fermented product;
(2) treating the *Monascus* fermented product with acetone for three times, wherein the ratio of the *Monascus* fermented product to the acetone is 1:10 ∼ 1:50;
(3) elevating the concentration of the product obtained from the previous step by a process of decompress concentration in a specific temperature range, wherein the specific temperature range in step (3) is 40°C∼60°C ;
(4.1) adding the product obtained from step (3) to one silica gel column;
(4.2) adding a plurality of washing solutions to the silica gel column sequentially, wherein the plurality of washing solutions in step (4.2) are Hexane, Hexane : Ethanol = 9:1, Hexane : Ethanol = 8:2, Hexane :Ethanol = 7:3, Hexane : Ethanol = 1:1, and Ethanol sequentially;
(4.3) collecting 12∼15 fractions flowing out from the silica gel column;
(4.4) analyzing the fractions collected in the previous step by one high performance liquid chromatography in combination with one photodiode-array (HPLC-PDA), and collecting fractions containing monascin and ankaflavin; and
(4.5) mixing the fractions collected in the previous step to form a pigment fraction;
(5) separating a yellow pigment fraction from the pigment fraction by a Sephadex LH-20 column chromatography;
(6.1) adding the yellow pigment fraction obtained from step (5) to one silica gel column;
(6.2) adding a plurality of washing solutions to the silica gel column sequentially, wherein the plurality of washing solutions in step (6.2) are Dichloromethane : Ethanol = 95:5, Dichloromethane : Ethanol = 9:1, and Dichloromethane : Ethanol = 4:1 sequentially;
(6.3) collecting 3-5 fractions flowing out from the silica gel column;
(6.4) analyzing the fractions collected in the previous step by one HPLC-PDA, and collecting fractions containing monascin and ankaflavin;
(6.5) mixing the fractions collected in the previous step to form a specific fraction containing monascin and ankaflavin;
(7.1) washing and separating the specific fraction containing monascin and ankaflavin obtained from step (6) by a C₁₈ column in combination with one high performance liquid chromatography (HPLC) and a washing solution, wherein the washing solution in step (7.1) is Methanol : water = 85:15;
(7.2) collecting 2 fractions flowing out from the C₁₈ column; and
(7.3) analyzing the fractions collected in the previous step by one HPLC-PDA, and collecting fractions containing ankaflavin;
(8) mixing the monascin and the ankaflavin according to a specific ratio, wherein the specific ratio in step (8) is in a range from 1:1 to 10:1, and the optimum value of the specific ratio in step (8) is 3.56:1.

6. The method for manufacturing the composition for lowering blood lipid and elevating high-density lipoprotein according to claim 5, wherein the composition comprises monascin and ankaflavin, which are both *Monascus* yellow pigments and extracted from a *Monascus* fermented product, wherein the content ratio of the monascin to the ankaflavin is in a range from 1:1 to 10:1.

7. The method for manufacturing the composition for lowering blood lipid and elevating high-density lipoprotein according to claim 6, wherein the optimum content ratio of the monascin to the ankaflavin is 3.56:1.

8. The method for manufacturing the composition for lowering blood lipid and elevating high-density lipoprotein according to claim 5, wherein the step (5) further comprises the steps of:
(5.1) adding the pigment fraction obtained from step (4) to one Sephadex LH-20 column;
(5.2) adding a washing solution to the Sephadex LH-20 column;
(5.3) collecting 3-5 fractions flowing out from the Sephadex LH-20 column;
(5.4) analyzing the fractions collected in the previous step by one HPLC-PDA, and collecting fractions containing monascin and ankaflavin; and
(5.5) mixing the fractions collected in the previous step to form the yellow pigment fraction;
wherein the washing solution in step (5.2) is Methanol : Acetonitrile = 9:1.

## Patentansprüche

1. Verfahren zur Herstellung einer Zusammensetzung zum Senken des Blutfetts und zum Erhöhen des hochdichten Lipoproteins, wobei das Verfahren folgende Schritte umfasst:
(1) Bereitstellen eines *Monascus-fermentierten* Produktes;
(2) dreimaliges Behandeln des *Monascus-*fermentierten Produktes mit Aceton, wobei das Verhältnis des *Monascus-fermentierten* Produktes zum Aceton 1:10∼1:50 beträgt;
(3) Erhöhen der Konzentration des aus dem vorherigen Schritt erhaltenen Produktes durch ein Verfahren zum Dekomprimieren der Konzentration in einem spezifischen Temperaturbereich, wobei der spezifische Temperaturbereich 40 °C∼60 °C ist;
(4.1) Zugeben des aus Schritt (3) erhaltenen Produktes zu einer Kieselgelsäule;
(4.2) sequentielles Zugeben mehrerer Waschlösungen zu der Kieselgelsäule, wobei die mehreren Waschlösungen in Schritt (4.2) sequentiell Hexan, Hexan:Ethanol=9:1, Hexan:Ethanol=8:2, Hexan:Ethanol=7:3, Hexan:Ethanol=1:1 und Ethanol sind;
(4.3) Sammeln von 12∼15 Fraktionen, die aus der Kieselgelsäule fließen;
(4.4) Analysieren der im vorherigen Schritt gesammelten Fraktionen durch Hochleistungsflüssigkeitschromatographie in Kombination mit einer Fotodioden-Anordnung (HPLC-PDA) und Sammeln von Fraktionen, die Monascin und Ankaflavin enthalten;
(4.5) Mischen der im vorherigen Schritt gesammelten Fraktionen zum Bilden einer Pigmentfraktion;
(5) Trennen einer gelben Pigmentfraktion von der Pigmentfraktion durch Sephadex LH-20 Säulenchromatographie;
(6.1) Zugeben der aus Schritt (5) erhaltenen gelben Pigmentfraktion zu einer Kieselgelsäule;
(6.2) sequentielles Zugeben mehrerer Waschlösungen zu der Kieselgelsäule, wobei die mehreren Waschlösungen in Schritt (6.2) sequentiell Dichlormethan:Ethanol=95:5, Dichlormethan:Ethanol=9:1 und Dichlormethan:Ethanol=4:1 sind;
(6.3) Sammeln von 3∼5 Fraktionen, die aus der Kieselgelsäule fließen;
(6.4) Analysieren der im vorherigen Schritt gesammelten Fraktionen durch HPLC-PDA und Sammeln von Fraktionen, die Monascin und Ankaflavin enthalten;
(6.5) Mischen der im vorherigen Schritt gesammelten Fraktionen zum Bilden eine spezifischen Fraktion, die Monascin und Ankaflavin enthält;
(7.1) Waschen und Trennen der aus Schritt (6) erhaltenen spezifischen Fraktion, die Monascin und Ankaflavin enthält, durch eine C₁₈-Säule in Kombination mit Hochleistungsflüssigkeitschromatographie (HPLC) und einer Waschlösung, wobei die Waschlösung in Schritt (7.1) Methanol:Wasser=85:15 ist;
(7.2) Sammeln von 2 Fraktionen, die aus der C₁₈-Säule fließen; und
(7.3) Analysieren der im vorherigen Schritt gesammelten Fraktionen durch HPLC-PDA und Sammeln von Fraktionen, die Monascin enthalten.

2. Verfahren zur Herstellung der Zusammensetzung zum Senken des Blutfetts und zum Erhöhen des hochdichten Lipoproteins nach Anspruch 1, wobei Schritt (5) ferner folgende Schritte umfasst:
(5.1) Zugeben der aus Schritt (4) erhaltenen Pigmentfraktion zu einer Sephadex LH-20 Säule;
(5.2) Zugeben einer Waschlösung zu der Sephadex LH-20 Säule;
(5.3) Sammeln von 3-5 Fraktionen, die aus der Sephadex LH-20 Säule fließen;
(5.4) Analysieren der im vorherigen Schritt gesammelten Fraktionen durch HPLC-PDA und Sammeln von Fraktionen, die Monascin und Ankaflavin enthalten; und
(5.5) Mischen der im vorherigen Schritt gesammelten Fraktionen zum Bilden der gelben Pigmentfraktion; wobei die Waschlösung in Schritt (5.2) Methanol:Acetonitril=9:1 ist.

3. Verfahren zur Herstellung einer Zusammensetzung zum Senken des Blutfetts und zum Erhöhen des hochdichten Lipoproteins, wobei das Verfahren folgende Schritte umfasst:
(1) Bereitstellen eines Monascus-fermentierten Produktes;
(2) dreimaliges Behandeln des *Monascus-*fermentierten Produktes mit Aceton, wobei das Verhältnis des *Monascus*-fermentierten Produktes zum Aceton 1:10∼1:50 beträgt;
(3) Erhöhen der Konzentration des aus dem vorherigen Schritt erhaltenen Produktes durch ein Verfahren zum Dekomprimieren der Konzentration in einem spezifischen Temperaturbereich, wobei der spezifische Temperaturbereich 40 °C∼60 °C ist;
(4.1) Zugeben des aus Schritt (3) erhaltenen Produktes zu einer Kieselgelsäule;
(4.2) sequentielles Zugeben mehrerer Waschlösungen zu der Kieselgelsäule, wobei die mehreren Waschlösungen in Schritt (4.2) sequentiell Hexan, Hexan:Ethanol=9:1, Hexan:Ethanol=8:2, Hexan:Ethanol=7:3, Hexan:Ethanol=1:1 und Ethanol sind;
(4.3) Sammeln von 12∼15 Fraktionen, die aus der Kieselgelsäule fließen;
(4.4) Analysieren der im vorherigen Schritt gesammelten Fraktionen durch Hochleistungsflüssigkeitschromatographie in Kombination mit einer Fotodioden-Anordnung (HPLC-PDA) und Sammeln von Fraktionen, die Monascin und Ankaflavin enthalten; und
(4.5) Mischen der im vorherigen Schritt gesammelten Fraktionen zum Bilden einer Pigmentfraktion;
(5) Trennen einer gelben Pigmentfraktion von der Pigmentfraktion durch Sephadex LH-20 Säulenchromatographie;
(6.1) Zugeben der aus Schritt (5) erhaltenen gelben Pigmentfraktion zu einer Kieselgelsäule;
(6.2) sequentielles Zugeben mehrerer Waschlösungen zu der Kieselgelsäule, wobei die mehreren Waschlösungen in Schritt (6.2) sequentiell Dichlormethan:Ethanol=95:5, Dichlormethan:Ethanol=9:1 und Dichlormethan:Ethanol=4:1 sind;
(6.3) Sammeln von 3-5 Fraktionen, die aus der Kieselgelsäule fließen;
(6.4) Analysieren der im vorherigen Schritt gesammelten Fraktionen durch HPLC-PDA und Sammeln von Fraktionen, die Monascin und Ankaflavin enthalten;
(6.5) Mischen der im vorherigen Schritt gesammelten Fraktionen zum Bilden eine spezifischen Fraktion, die Monascin und Ankaflavin enthält;
(7.1) Waschen und Trennen der aus Schritt (6) erhaltenen spezifischen Fraktion, die Monascin und Ankaflavin enthält, durch eine C₁₈-Säule in Kombination mit Hochleistungsflüssigkeitschromatographie (HPLC) und einer Waschlösung, wobei die Waschlösung in Schritt (7.1) Methanol:Wasser=85:15 ist;
(7.2) Sammeln von 2 Fraktionen, die aus der C₁₈-Säule fließen; und
(7.3) Analysieren der im vorherigen Schritt gesammelten Fraktionen durch HPLC-PDA und Sammeln von Fraktionen, die Ankaflavin enthalten.

4. Verfahren zur Herstellung der Zusammensetzung zum Senken des Blutfetts und zum Erhöhen des hochdichten Lipoproteins nach Anspruch 3, wobei Schritt (5) ferner folgende Schritte umfasst:
(5.1) Zugeben der aus Schritt (4) erhaltenen Pigmentfraktion zu einer Sephadex LH-20 Säule;
(5.2) Zugeben einer Waschlösung zu der Sephadex LH-20 Säule;
(5.3) Sammeln von 3∼5 Fraktionen, die aus der Sephadex LH-20 Säule fließen;
(5.4) Analysieren der im vorherigen Schritt gesammelten Fraktionen durch HPLC-PDA und Sammeln von Fraktionen, die Monascin und Ankaflavin enthalten; und
(5.5) Mischen der im vorherigen Schritt gesammelten Fraktionen zum Bilden der gelben Pigmentfraktion; wobei die Waschlösung in Schritt (5.2) Methanol:Acetonitril=9:1 ist.

5. Verfahren zur Herstellung einer Zusammensetzung zum Senken des Blutfetts und zum Erhöhen des hochdichten Lipoproteins, wobei das Verfahren folgende Schritte umfasst:
(1) Bereitstellen eines *Monascus*-fermentierten Produktes;
(2) dreimaliges Behandeln des *Monascus-*fermentierten Produktes mit Aceton, wobei das Verhältnis des *Monascus*-fermentierten Produktes zum Aceton 1:10-1:50 beträgt;
(3) Erhöhen der Konzentration des aus dem vorherigen Schritt erhaltenen Produktes durch ein Verfahren zum Dekomprimieren der Konzentration in einem spezifischen Temperaturbereich, wobei der spezifische Temperaturbereich in Schritt (3) 40 °C∼60 °C ist;
(4.1) Zugeben des aus Schritt (3) erhaltenen Produktes zu einer Kieselgelsäule;
(4.2) sequentielles Zugeben mehrerer Waschlösungen zu der Kieselgelsäule, wobei die mehreren Waschlösungen in Schritt (4.2) sequentiell Hexan, Hexan:Ethanol=9:1, Hexan:Ethanol=8:2, Hexan:Ethanol=7:3, Hexan:Ethanol=1:1 und Ethanol sind;
(4.3) Sammeln von 12∼15 Fraktionen, die aus der Kieselgelsäule fließen;
(4.4) Analysieren der im vorherigen Schritt gesammelten Fraktionen durch Hochleistungsflüssigkeitschromatographie in Kombination mit einer Fotodioden-Anordnung (HPLC-PDA) und Sammeln von Fraktionen, die Monascin und Ankaflavin enthalten ; und
(4.5) Mischen der im vorherigen Schritt gesammelten Fraktionen zum Bilden einer Pigmentfraktion;
(5) Trennen einer gelben Pigmentfraktion von der Pigmentfraktion durch Sephadex LH-20 Säulenchromatographie;
(6.1) Zugeben der aus Schritt (5) erhaltenen gelben Pigmentfraktion zu einer Kieselgelsäule;
(6.2) sequentielles Zugeben mehrerer Waschlösungen zu der Kieselgelsäule, wobei die mehreren Waschlösungen in Schritt (6.2) sequentiell Dichlormethan:Ethanol=95:5, Dichlormethan:Ethanol=9:1 und Dichlormethan:Ethanol=4:1 sind;
(6.3) Sammeln von 3∼5 Fraktionen, die aus der Kieselgelsäule fließen;
(6.4) Analysieren der im vorherigen Schritt gesammelten Fraktionen durch HPLC-PDA und Sammeln von Fraktionen, die Monascin und Ankaflavin enthalten;
(6.5) Mischen der im vorherigen Schritt gesammelten Fraktionen zum Bilden eine spezifischen Fraktion, die Monascin und Ankaflavin enthält;
(7.1) Waschen und Trennen der aus Schritt (6) erhaltenen spezifischen Fraktion, die Monascin und Ankaflavin enthält, durch eine C₁₈-Säule in Kombination mit Hochleistungsflüssigkeitschromatographie (HPLC) und einer Waschlösung, wobei die Waschlösung in Schritt (7.1) Methanol:Wasser=85:15 ist;
(7.2) Sammeln von 2 Fraktionen, die aus der C₁₈-Säule fließen; und
(7.3) Analysieren der im vorherigen Schritt gesammelten Fraktionen durch HPLC-PDA und Sammeln von Fraktionen, die Ankaflavin enthalten;
(8) Mischen des Monascins und des Ankaflavins gemäß eines spezifischen Verhältnisses, wobei das spezifische Verhältnis in Schritt (8) in einem Bereich von 1:1 bis 10:1 liegt und der optimale Wert des spezifischen Verhältnisses in Schritt (8) 3,56:1 beträgt.

6. Verfahren zur Herstellung der Zusammensetzung zum Senken des Blutfetts und zum Erhöhen des hochdichten Lipoproteins nach Anspruch 5, wobei die Zusammensetzung Monascin und Ankaflavin umfasst, welche beide gelbe *Monascus*-Pigmente sind und aus einem *Monascus-*fermentierten Produkt extrahiert sind, wobei das Verhältnis des Gehaltes von Monascin zu Ankaflavin im Bereich von 1:1 bis 10:1 liegt.

7. Verfahren zur Herstellung der Zusammensetzung zum Senken des Blutfetts und zum Erhöhen des hochdichten Lipoproteins nach Anspruch 6, wobei das optimale Verhältnis des Gehaltes von Monascin zu Ankaflavin 3,56:1 beträgt.

8. Verfahren zur Herstellung der Zusammensetzung zum Senken des Blutfetts und zum Erhöhen des hochdichten Lipoproteins nach Anspruch 5, wobei Schritt (5) ferner folgende Schritte umfasst:
(5.1) Zugeben der aus Schritt (4) erhaltenen Pigmentfraktion zu einer Sephadex LH-20 Säule;
(5.2) Zugeben einer Waschlösung zu der Sephadex LH-20 Säule;
(5.3) Sammeln von 3∼5 Fraktionen, die aus der Sephadex LH-20 Säule fließen;
(5.4) Analysieren der im vorherigen Schritt gesammelten Fraktionen durch HPLC-PDA und Sammeln von Fraktionen, die Monascin und Ankaflavin enthalten; und
(5.5) Mischen der im vorherigen Schritt gesammelten Fraktionen zum Bilden der gelben Pigmentfraktion; wobei die Waschlösung in Schritt (5.2) Methanol:Acetonitril=9:1 ist.

## Revendications

1. Procédé de fabrication d'une composition pour abaisser le niveau de lipides dans le sang et augmenter le niveau de lipoprotéines de haute densité, dans lequel le procédé comprend les étapes suivantes :
(1) fourniture d'un produit fermenté de *Monascus ;*
(2) traitement du produit fermenté de *Monascus* avec de l'acétone trois fois, dans lequel le rapport du produit fermenté de *Monascus* sur l'acétone est de 1/10 à 1/50 ;
(3) augmentation de la concentration du produit obtenu de l'étape précédente par un processus de concentration par décompression dans une plage de températures spécifique, dans lequel la plage de températures spécifique est de 40°C à 60°C ;
(4.1) ajout du produit obtenu de l'étape (3) à une colonne de gel de silice ;
(4.2) ajout d'une pluralité de solutions de lavage à la colonne de gel de silice séquentiellement, dans lequel la pluralité de solutions de lavage de l'étape (4.2) sont hexane, hexane/éthanol = 9/1, hexane/éthanol = 8/2, hexane/éthanol = 7/3, hexane/éthanol = 1/1, et éthanol, séquentiellement ;
(4.3) collecte de 12 à 15 fractions s'écoulant de la colonne de gel de silice ;
(4.4) analyse des fractions collectées dans l'étape précédente par une chromatographie en phase liquide haute performance couplée à un détecteur à barrette de photodiodes (CLHP-PDA), et collecte des fractions contenant la monascine et l'ankaflavine ;
(4.5) mélange des fractions collectées dans l'étape précédente pour former une fraction pigment ;
(5) séparation d'une fraction pigment jaune de la fraction pigment par chromatographie sur colonne Sephadex LH-20 ;
(6.1) ajout de la fraction pigment jaune obtenue de l'étape (5) à une colonne de gel de silice ;
(6.2) ajout d'une pluralité de solutions de lavage à la colonne de gel de silice séquentiellement, dans lequel la pluralité de solutions de lavage de l'étape (6.2) sont dichlorométhane/éthanol = 95/5, dichlorométhane/éthanol = 9/1, et dichlorométhane/éthanol = 4/1, séquentiellement ;
(6.3) collecte de 3 à 5 fractions s'écoulant de la colonne de gel de silice ;
(6.4) analyse des fractions collectées dans l'étape précédente par CLHP-PDA, et collecte des fractions contenant la monascine et l'ankaflavine ; et
(6.5) mélange des fractions collectées dans l'étape précédente pour former une fraction spécifique contenant la monascine et l'ankaflavine ;
(7.1) lavage et séparation de la fraction spécifique contenant la monascine et l'ankaflavine obtenue de l'étape (6) par une colonne C₁₈ couplée à une chromatographie en phase liquide haute performance (CLHP) et une solution de lavage, dans lequel la solution de lavage de l'étape (7.1) est méthanol/eau = 85/15 ;
(7.2) collecte de 2 fractions s'écoulant de la colonne C₁₈ ; et
(7.3) analyse des fractions collectées dans l'étape précédente par CLHP-PDA, et collecte des fractions contenant la monascine.

2. Procédé de fabrication de la composition pour abaisser le niveau de lipides dans le sang et augmenter le niveau de lipoprotéines de haute densité selon la revendication 1, dans lequel l'étape (5) comprend en outre les étapes suivantes :
(5.1) ajout de la fraction pigment obtenue de l'étape (4) à une colonne Sephadex LH-20 ;
(5.2) ajout d'une solution de lavage à la colonne Sephadex LH-20 ;
(5.3) collecte de 3 à 5 fractions s'écoulant de la colonne Sephadex LH-20 ;
(5.4) analyse des fractions collectées dans l'étape précédente par CLHP-PDA, et collecte des fractions contenant la monascine et l'ankaflavine ; et
(5.5) mélange des fractions collectées dans l'étape précédente pour former la fraction pigment jaune ;
dans lequel la solution de lavage de l'étape (5.2) est du méthanol/acétonitrile = 9/1.

3. Procédé de fabrication d'une composition pour abaisser le niveau de lipides dans le sang et augmenter le niveau de lipoprotéines de haute densité, dans lequel le procédé comprend les étapes suivantes :
(1) fourniture d'un produit fermenté de *Monascus ;*
(2) traitement du produit fermenté de *Monascus* avec de l'acétone trois fois, dans lequel le rapport du produit fermenté de *Monascus* sur l'acétone est de 1/10 à 1/50 ;
(3) augmentation de la concentration du produit obtenu de l'étape précédente par un processus de concentration par décompression dans une plage de températures spécifique, dans lequel la plage de températures spécifique est de 40°C à 60°C ;
(4.1) ajout du produit obtenu de l'étape (3) à une colonne de gel de silice ;
(4.2) ajout d'une pluralité de solutions de lavage à la colonne de gel de silice séquentiellement, dans lequel la pluralité de solutions de lavage de l'étape (4.2) sont hexane, hexane/éthanol = 9/1, hexane/éthanol = 8/2, hexane/éthanol = 7/3, hexane/éthanol = 1/1, et éthanol, séquentiellement ;
(4.3) collecte de 12 à 15 fractions s'écoulant de la colonne de gel de silice ;
(4.4) analyse des fractions collectées dans l'étape précédente par une chromatographie en phase liquide haute performance couplée à un détecteur à barrette de photodiodes (CLHP-PDA), et collecte des fractions contenant la monascine et l'ankaflavine ; et
(4.5) mélange des fractions collectées dans l'étape précédente pour former une fraction pigment ;
(5) séparation d'une fraction pigment jaune de la fraction pigment par une chromatographie sur colonne Sephadex LH-20 ;
(6.1) ajout de la fraction pigment jaune obtenue de l'étape (5) à une colonne de gel de silice ;
(6.2) ajout d'une pluralité de solutions de lavage à la colonne de gel de silice séquentiellement, dans lequel la pluralité de solutions de lavage de l'étape (6.2) sont dichlorométhane/éthanol = 95/5, dichlorométhane/éthanol = 9/1, et dichlorométhane/éthanol = 4/1, séquentiellement ;
(6.3) collecte de 3 à 5 fractions s'écoulant de la colonne de gel de silice ;
(6.4) analyse des fractions collectées dans l'étape précédente par CLHP-PDA, et collecte des fractions contenant la monascine et l'ankaflavine ;
(6.5) mélange des fractions collectées dans l'étape précédente pour former une fraction spécifique contenant la monascine et l'ankaflavine ;
(7.1) lavage et séparation de la fraction spécifique contenant la monascine et l'ankaflavine obtenue de l'étape (6) par une colonne C₁₈ couplée à une chromatographie en phase liquide haute performance (CLHP) et une solution de lavage, dans lequel la solution de lavage de l'étape (7.1) est méthanol/eau = 85/15 ;
(7.2) collecte de 2 fractions s'écoulant de la colonne C₁₈ ; et
(7.3) analyse des fractions collectées dans l'étape précédente par CLHP-PDA, et collecte des fractions contenant l'ankaflavine.

4. Procédé de fabrication de la composition pour abaisser le niveau de lipides dans le sang et augmenter le niveau de lipoprotéines de haute densité selon la revendication 3, dans lequel l'étape (5) comprend en outre les étapes suivantes :
(5.1) ajout de la fraction pigment jaune obtenue de l'étape (4) à une colonne Sephadex L-20 ;
(5.2) ajout d'une solution de lavage à la colonne Sephadex LH-20 ;
(5.3) collecte de 3 à 5 fractions s'écoulant de la colonne Sephadex LH-20 ;
(5.4) analyse des fractions collectées dans l'étape précédente par CLHP-PDA, et collecte des fractions contenant la monascine et l'ankaflavine ; et
(5.5) mélange des fractions collectées dans l'étape précédente pour former la fraction pigment jaune ;
dans lequel la solution de lavage de l'étape (5.2) est méthanol/acétonitrile = 9/1.

5. Procédé de fabrication d'une composition pour abaisser le niveau de lipides dans le sang et augmenter le niveau de lipoprotéines de haute densité, dans lequel le procédé comprend les étapes suivantes :
(1) fourniture d'un produit fermenté de *Monascus ;*
(2) traitement du produit fermenté de *Monascus* avec de l'acétone trois fois, dans lequel le rapport du produit fermenté de *Monascus* sur l'acétone est de 1/10 à 1/50 ;
(3) augmentation de la concentration du produit obtenu dans l'étape précédente par un processus de concentration par décompression dans une plage de températures spécifique, dans lequel la plage de températures spécifique dans l'étape (3) est de 40°C à 60°C ;
(4.1) ajout du produit obtenu de l'étape (3) à une colonne de gel de silice ;
(4.2) ajout d'une pluralité de solutions de lavage à la colonne de gel de silice séquentiellement, dans lequel la pluralité de solutions de lavage de l'étape (4.2) sont hexane, hexane/éthanol = 9/1, hexane/éthanol = 8/2, hexane/éthanol = 7/3, hexane/éthanol = 1/1, et éthanol, séquentiellement ;
(4.3) collecte de 12 à 15 fractions s'écoulant de la colonne de gel de silice ;
(4.4) analyse des fractions collectées dans l'étape précédente par une chromatographie en phase liquide haute performance couplée à un détecteur à barrette de photodiodes (CLHP-PDA), et collecte des fractions contenant la monascine et l'ankaflavine ; et
(4.5) mélange des fractions collectées dans l'étape précédente pour former une fraction pigment ;
(5) séparation d'une fraction pigment jaune de la fraction pigment par une chromatographie sur colonne Sephadex LH-20 ;
(6.1) ajout de la fraction pigment jaune obtenue de l'étape (5) à une colonne de gel de silice ;
(6.2) ajout d'une pluralité de solutions de lavage à la colonne de gel de silice séquentiellement, dans lequel la pluralité de solutions de lavage de l'étape (6.2) sont dichlorométhane/éthanol = 95/5, dichlorométhane/éthanol = 9/1, et dichlorométhane/éthanol = 4/1, séquentiellement ;
(6.3) collecte de 3 à 5 fractions s'écoulant de la colonne de gel de silice ;
(6.4) analyse des fractions collectées dans l'étape précédente par CLHP-PDA, et collecte des fractions contenant la monascine et l'ankaflavine ;
(6.5) mélange des fractions collectées dans l'étape précédente pour former une fraction spécifique contenant la monascine et l'ankaflavine ;
(7.1) lavage et séparation de la fraction spécifique contenant la monascine et l'ankaflavine obtenue de l'étape (6) par une colonne C₁₈ couplée à une chromatographie en phase liquide haute performance (CLHP) et une solution de lavage, dans lequel la solution de lavage de l'étape (7.1) est méthanol/eau = 85/15 ;
(7.2) collecte de 2 fractions s'écoulant de la colonne C₁₈ : et
(7.3) analyse des fractions collectées dans l'étape précédente par CLHP-PDA, et collecte des fractions contenant l'ankaflavine ;
(8) mélange de la monascine et de l'ankaflavine selon un rapport spécifique, dans lequel le rapport spécifique dans l'étape (8) se trouve dans une page de 1/1 à 10/1, et la valeur optimale du rapport spécifique dans l'étape (8) est de 3,56/1.

6. Procédé de fabrication d'une composition pour abaisser le niveau de lipides dans le sang et augmenter le niveau de lipoprotéines de haute densité selon la revendication 5, dans lequel la composition comprend de la monascine et de l'ankaflavine, qui sont toutes les deux des pigments jaunes de *Monascus* et extraites d'un produit fermenté de *Monascus,* dans lequel le rapport de teneur de la monascine sur l'ankaflavine se trouve dans une plage de 1/1 à 10/1.

7. Procédé de fabrication de la composition pour abaisser le niveau de lipides dans le sang et augmenter le niveau de lipoprotéines de haute densité selon la revendication 6, dans lequel le rapport de teneur optimal de la monascine sur l'ankaflavine est de 3,56/1.

8. Procédé de préparation de la composition pour abaisser le niveau de lipides dans le sang et augmenter le niveau de lipoprotéines de haute densité selon la revendication 5, dans lequel l'étape (5) comprend en outre les étapes suivantes :
(5.1) ajout de la fraction pigment jaune obtenue de l'étape (4) à une colonne Sephadex LH-20 ;
(5.2) ajout d'une solution de lavage à la colonne Sephadex LH-20 ;
(5.3) collecte de 3 à 5 fractions s'écoulant de la colonne Sephadex LH-20 ;
(5.4) analyse des fractions collectées dans l'étape précédente par CLHP-PDA, et collecte des fractions contenant la monascine et l'ankaflavine ; et
(5.5) mélange des fractions collectées dans l'étape précédente pour former la fraction pigment jaune ;
dans lequel la solution de lavage de l'étape (5.2) est du méthanol/acétonitrile = 9/1.
